# EUROPEAN PATENT SPECIFICATION

(11) **EP 3 649 922 B1**
(45) Date of publication and mention of the grant of the patent: **11.03.2026**
(21) Application number: 19151503.0
(22) Date of filing: 11.01.2019
(51) Int. Cl.: A61B 3/125, A61B 3/117, A61B 3/103, A61B 3/028

(54) **DEVICE FOR COMPENSATING PHYSICAL AND/OR OPTICAL DISTORTIONS OF A HUMAN EYE**
VORRICHTUNG ZUR KOMPENSATION VON PHYSIKALISCHEN UND/ODER OPTISCHEN VERZERRUNGEN EINES MENSCHLICHEN AUGES
DISPOSITIF DE COMPENSATION DE DISTORSIONS OPTIQUES ET/OU PHYSIQUES D'UN IL HUMAIN

(30) Priority: 12.11.2018 EP 18205645
(43) Date of publication of application: 13.05.2020
(73) Proprietor: Ellex Medical PTY Ltd, Adelaide, SA 5000 (AU)
(72) Inventor: Edwards, Howard Joseph, Mawson Lakes, South Australia 5095 (AU)
(74) Representative: Sattler de Sousa e Brito, Clara

(56) References cited:
- FR-A- 1 175 511
- JP-A- 2011 062 325
- US-A- 4 598 984
- US-A- 4 664 490
- US-A- 5 200 773

## Description

The present invention relates to a device for compensating physical and/or optical distortions of a human eye comprising a housing and a lens arranged in an inner space of the housing.

In ophthalmology, lasers are projected onto a retina of a human eye to treat retinal pathologies. They are commonly used to treat diabetic retinopathy, proliferative diabetic oedema, anastomosis, vitreolysis and other retina diseases. In recent years, sub-threshold laser treatments have emerged to treat retinal diseases with aim of less collateral damage to the neural retina than supra threshold treatments.

By performing a super threshold treatment there is a rapid feedback due to visible tissue interaction. Induced physical and colour changes that are visible during treatment so that the skilled surgeon can compensate for beam distortions that tend to increase spot size by incrementally increasing power as required.

With sub-threshold laser treatment, as there is no immediately observable visual feedback, so the surgeon cannot compensate for these distortions hence inconsistent treatment occurs. This is especially pronounced in the inferior hemisphere of the eye compared with the superior hemisphere.

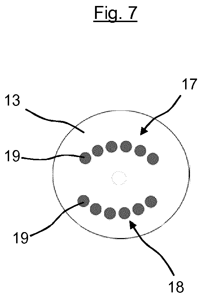

Diagnostic lenses for indirect ophthalmoscopy are used by ophthalmologists and optometrists to observe the fundus of an eye for diagnostic or surgical procedures. For example, document US 5 200 773 A describes a diagnostic indirect ophthalmoscopy lens device utilized for illumination and observation of the fundus of the eye which may include a plus powered aspheric contact element and at least one anterior lens element which may also have aspheric surfaces and/or positive refractive power. Document US 5 200 773 A also describes a reinverting prism. In figure 7, the prism and the lenses are shown, wherein the lenses are positioned close to the eye and the prism is positioned at the opposite end of the device. In figure 8, an additional lens is shown, through which the light enters the device.

Further prior art lens devices are known that are routinely used to facilitate ophthalmoscopy during retinal treatment. These lens devices are temporarily placed on the cornea and a) keep the eye from moving, b) create a desired field of view c) create the desired magnification and d) prevent the blink reflex from interfering with the treatment. Lens device magnification and a field of view of the lens device are critical parameters for visualizing and for treating a certain retinal pathology.

A problem in achieving of good treatment results is that the visual axis of the human eye is misaligned relative to the optical axis of the eye. This difference is described by the Liou and Brennen model of the human eye. In the following, several values are mentioned that apply to an average human eye.
- (01): The Spherical Aberration of the cornea is from +0.2 µm and +0.4 µm,
- (02): Eye's optics are not well aligned,
- (03): Visual axis is not collinear with optical axis: tilt 5° in nasal
- (04): The pupil is not centred on the optical axis: decentration of 0.5 mm in nasal,
- (05): The visual axis does not pass through the centre of the pupil or the centre of lens: decentration of an average approximation of 0.34 mm,
- (06): Crystalline is 0.2° tilted in temporal,
- (07): Crystalline is decentred from 0.1 mm in nasal,
- (08): Fovea is at 5.7° from the optic axis,
- (09): Averagely +0.1 µm Spherical Aberration remains.

A further problem in achieving good treatment results is that the optical zone of the lens device usually does not cover all relevant part of the retina. Therefore, the surgeon tilts the contact lens off the optical axis of the human eye in order to treat and visualize e.g. parts of the inferior hemisphere and nasal or temporal quadrants of the retina, depending on being a left or right eye. However, tilting the lens device in any orientation introduces unwanted high order aberrations which will deform the laser light.

The aforementioned factors, in particular the normal physical and optical disturbances of the human eye and the tilting of the lens device in the treatment, influence the laser spot fluence. However, preserving laser spot fluence is critical to accurate dosimetry what is important for the treatment success.

An object of the present invention is to provide a device with which better treatment success can be achieved.

The object is solved by the aforementioned device that is characterized in that the device comprises a prism arranged in the inner space.

The device comprises the advantage that the prism corrects the misalignment of the visual axis relative to the optical axis. In particular, the prism is formed such that it compensates the misalignment between the visual axis of the human eye and the optical axis of the human eye. This results in that laser spots of uniform diameter and/or radiance can be achieved when projecting a laser into the eye for therapeutic treatment. Thus, also laser spot images of uniform diameter and/or radiance can be achieved. Further, it is possible to preserve laser spot fluence. Thus, a device is provided with which natural physical and/or optical distortions of the human eye are compensated and a magnification of the visualized part of the human eye can be realized.

A prism is an optical component that disperses and/or reflects light. The prism can have a triangular base area. Alternativately, the prism can have an n-sided base area (n is an integer number) like e.g. a rectangle base area.

According to an embodiment the lens is arranged at an end of the housing. The end can be part of housing that is distal to the human eye. In particular, the lens can be arranged such in the inner space that the laser light enters the device via the lens. Thus, a simply constructed device is provided.

The lens can be an aspheric lens. This shows the advantage that the lens compensates the distortion due to the spherical aberration of the cornea as mentioned before in item 1 of the Liou and Brennen model.

According to an embodiment the device comprises a further lens. The further lens is arranged at a further end of the housing. The further end can be a part of the housing that is arranged distal to the human eye. The further lens is arranged such that it can be brought in direct contact with the cornea of the human eye, in particular for treatment. The further lens is arranged such that the laser light entered the device leaves the device via the further lens.

During treatment of the human eye the cornea adapts to the base curve of the further lens. This shows the advantage that the optical distortions mentioned above in items 4 and 5 of the Liou and Brennen model can be avoided due to the provision of the further lens.

The prism is arranged between the lens and the further lens. In particular, the prism can be arranged between the lens and the further lens along an optical axis of the device. The prism and the further lens and/or the lens are arranged at distance from each other. As a result, a simply constructed device can be provided.

In an alternative embodiment the prism is attached to the further lens. This embodiment shows the advantage that internal reflection of laser light entered the device is reduced. Further, such embodiment shows the advantage that cost can be saved in comparison to the embodiment at which the prism and the further lens are arranged at distance from each other. The prism can be directly attached to the further lens, that means, no other components are arranged between the prism and the further lens.

According to an embodiment a further prism is arranged in the inner space. The further prism can be arranged between the prism and the lens. In particular, the further prism can be arranged between the prism and the lens along the optical axis of the device. Provision of the further prism shows the advantage that the distortions mentioned above in items 3, 6 and 7 of the Liou and Brennen model can be corrected. As a result, a device is provided that compensates the natural physical and/or optical distortions of the human eye.

The device discussed above has the advantage that the optical zone of the device is greater than an optical zone of the lens devices known from the prior art. Further, the optical zone of the device can be shifted in comparison to the optical zone of the known lens devices. This is achieved by the two prisms arranged in the housing. In particular, the optical zone of the device can be shifted to the vascular arcade zone. That means, it is not necessary anymore to tilt the device in order to treat and/or visualize the vascular arcade zone of the human eye. Further, high order aberrations are compensated by the device. Thus, the eye treatment is simplified and more accurate than in the prior art.

The optical zone of the device defines the area of the human eye, in particular the retina, that can be treated and visualized by the device. The greater the optical zone the more area of the human eye can be treated and visualized.

The device has also the advantage that it keeps the human eye from moving and as mentioned before creates a desired optical zone. Further, the device prevents the blink reflex from interfering with the treatment and magnifies the watched part of the retina.

The prism and the further prism can be moved with respect to each other and/or in that the prism and the further prism can be rotated with respect to each other. These embodiments have the advantage that the optical properties of the device can be changed. This is necessary because the factors mentioned above in items 1 to 9 of the Liou and Brennen model that influence the distortion of the human eye are not constant for each human eye. That means human eyes can differ from each other in some of that factors. Further, the factors are dependent of the age of the human person. As a result, due to movement of the prism and the further prism relative to another the device can be easily set to the human eye to be treated. That means, one device can be used in different human eyes that differ from each other in at least one of the items 1 to 9 mentioned above. In other words, the device can compensate the natural physical and/or optical distortions of several eyes.

According to an embodiment the lens and/or the further lens can be releasably connected with the housing, respectively. Further, the prism and/or the further prism can be releasably connected with the housing, respectively. This shows the advantage that the lens and/or the further lens and/or prism and/or further prism can easily be exchanged. The exchange can be necessary in order to adapt the device on the human eye to be treated.

The lens can be exchanged by another lens from a plurality of another lenses, wherein the another lenses differ from each other in at least one optical property. The further lens can be exchanged by another further lens from a plurality of another further lenses, wherein the another further lenses differ from each other in at least one optical property.

Further, the prism can be exchanged by another prism from a plurality of another prisms, wherein the another prisms differ from each other in at least one optical property. Alternatively or additionally, the further prism can be exchanged by another further prism from a plurality of another further prisms, wherein the another further prisms differ from each other in at least one optical property.

The surgeon can choose from the plurality of the another lenses and/or the another further lenses the lens or lenses having the optical properties to compensate at least some of the natural physical and/or optical distortions of the respective human eye. Additionally or alternatively, the surgeon can choose from the plurality of the another prisms and/or the another further prisms the prism or prisms having the optical properties to compensate at least some of the natural physical and/or optical distortions of the respective human eye

The prism and the further prism can be connected together to be modular. Further, the prism and the further lens can be connected together to be modular. The modular connection enables a simple removal of the prism, the further prism and/or the further lens because only one component has to be removed from the device. This shows the advantage that the removal of the device parts is simplified.

According to an embodiment the housing has a cylindrical portion comprising the lens and a truncated portion comprising the prism. Thus, the device is simply constructed.

According to an independent aspect of the invention a method for treatment of a human eye comprising the following steps is provided:
▪ Placing a device for compensating physical and optical distortions, in particular a device as described above and/or according to one of the claims 1 to 14 mentioned below, on a cornea of a human eye,
▪ Exposing the human eye, in particular a part of a retina of the human eye, with laser light,
▪ Considering the exposed portion of the human eye by means of the device.

The method steps can be performed in the aforementioned sequence.

The device can be placed colinear to the optical axis of the human eye. In particular, the device is arranged such that an optical axis of the device is collinear with the optical axis of the human eye.

A laser providing the laser light can be arranged such that the laser light passes through the device and afterwards into the human eye. The laser con be controlled such that several laser spots are generated in the human eye, in particular in the retina. The laser spots differ in distance in vertical and/or horizontal direction from each other.

The human eye can be treated by usage of the device with a super threshold treatment and/or with a sub-threshold treatment. In the super-threshold treatment a rapid feedback is received due to visible tissue interaction. In contrary to that in the sub-threshold treatment no immediate visual feedback is received due to tissue interaction.

The device can be used for treatment of eye disease. In particular, the device can be a laser contact lens comprising the aforementioned structure for treatment of eye disease.

According to a further embodiment the device can comprise an optical filter for blocking light of a specific wavelength. Further, the device, in particular parts of the device, can comprise a filter coating for blocking light of a specific wavelength. The optical filter and/or the filter coating can be adapted to block violet light having a wavelength between 400-440 nm and to block blue light having a wavelength between 440-500 nm. The optical filter and/or the filter coating provide good photo-protection to the retina from illumination light sources that produce shorter wavelengths causing potential damage to the retina.

A particular advantage of the invention is to use the device for the correction of ametropia. In particular, the optical design of the device can be used for intraocular lenses, contact lenses and spectacle lenses in the correction of ametropia. Further, the device can be used for retina treatment.

The subject matter of the invention is illustrated by way of example and schematically in the drawing, and will be described below on the basis of the figures, wherein identical elements or elements of identical action are normally denoted by the same reference designations. In the drawings:
Figure 1: shows a standard human eye,
Figure 2: shows a lens device known from the prior art placed on the human eye shown in figure 1,
Figure 3: shows the laser spots visible with the lens device shown in figure 2 after laser treatment,
Figure 4: shows a device according to a first embodiment of the invention,
Figure 5: shows a device according to a second embodiment of the invention,
Figure 6: shows the inventive device placed on a human eye as shown in figure 1,
Figure 7: shows the laser spots visible with the device shown in figure 6.

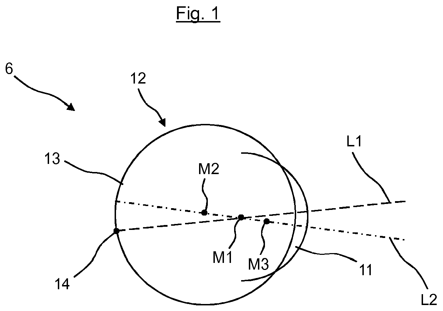

Figure 1 shows a schematic view of a standard emmetropic human eye 6. The human eye 6 comprises a cornea 11 of the curvature of the cornea 11 and an eyeball 12. A centre M1 of the curvature of the cornea 11 and a centre M2 of the eyeball 12 are also shown in figure 1. Figure 1 shows also a centre M3 of a pupil non-shown in figure 1. The cornea 11 is provided in the front part of the human eye 6. The eyeball 12 comprises a retina 13 provided at the rear part of the human eye 6. The human eye 6 also comprises a fovea 14.

A visual axis L1 of the human eye 6 passes through the fovea 14 and the centre M1 of the curvature of cornea 11. An optical axis L2 of the human eye 6 passes through the centre M3 of pupil and the centre M2 of eyeball 12. As is evident from figure 1 the visual axis L1 and the optical axis L2 are not aligned with respect to each other.

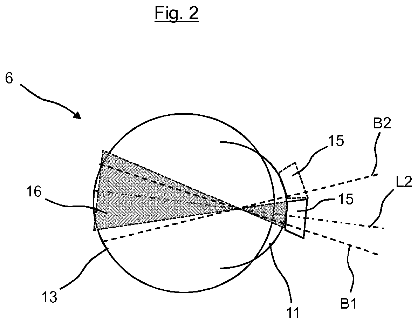

Figure 2 shows a lens device 15 known from the prior art. The lens device 15 is placed on the cornea 11. In figure 2 the optical zone 16 of the lens device 15 is shown. The optical zone 16 defines the area of the retina 13 that can be treated, in particular with reasonable uniform laser spots, by the non-shown laser and, thus, visualized by the lens device 15.

In the treatment the retina 13 is exposed with two laser beams B1, B2. The laser beams B1, B2 differ in orientation from each other. A first laser beam B1 is orientated such that it exposes a region of the retina 13 that is arranged within the optical zone 16. A second laser beams L2 is orientated such that it exposes a region of the retina 13 that is arranged outside, in particular an acceptable distortion free, optical zone, 16 of the lens device 15.

In order to expose and/or visualize the region of the retina 13 exposed by the second laser beam B2 the lens device 15 has to be tilted to a different position. The tilted lens device 15 is shown in figure 2 in dotted lines. However, in the tilted position the lens device 15 is not arranged collinear to the optical axis L2 of the human eye 6.

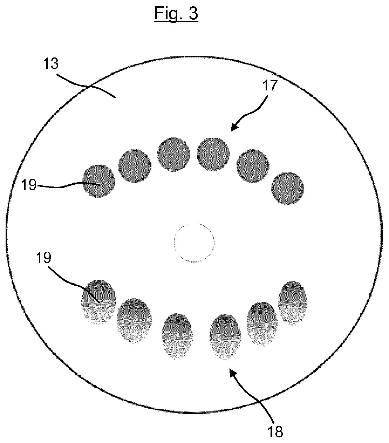

Figure 3 shows several laser spots 19 resulting from exposing the retina 13 by the first and second laser beams B1, B2. The laser spots 19 arranged in the upper arcade 17 result from exposing regions of the retina 13 with first laser beams B1. The laser spots 19 arranged in the lower arcade 18 result from exposing regions of retina 13 with second laser beams L2. As can be seen from figure 3 the lower arcade 18 shows irregular laser spots 19. In particular, the spot sizes increase in the lower arcade 18.

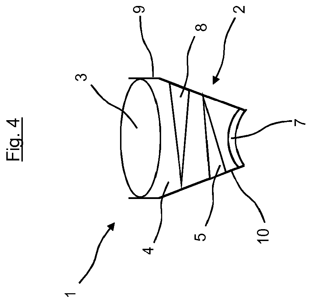

Figure 4 shows a device 1 for compensating physical and/or optical distortions of the human eye 6 according to a first embodiment. The device 1 comprises a housing 2 and a lens 3 arranged in an inner space 4 of the housing 2. Further, the device 1 comprises a prism 5 arranged in the inner space 4. The prism 5 is formed such that it compensates the misalignment between the visual axis L1 and the optical axis L2 both shown in figure 1. The prism 5 has a triangular shaped base area.

Additionally, the device 1 comprises a further lens 7 and a further prism 8. The further prism 8 has also a triangular shaped base area. The lens 3 is arranged at an end of the housing 2 distal to the human eye 6. In usage of the device 1 for an eye treatment laser light coming from the non-shown laser enters the device 1 via the lens 3 and leaves it via the further lens 7. The lens 3 is an aspheric lens.

The further lens 7 is arranged at a further end of the housing 2. In particular, the further lens 7 is arranged such that in usage of the device 1 for an eye treatment the further lens 7 comes in direct contact with the cornea 11.

The prism 5 is arranged between the lens 3 and the further lens 7. The further prism 8 is arranged between the prism 5 and the lens 3. The prism 5 is arranged at distance from the further lens 7 and the further prism 8.

The housing 2 comprises a cylindrical portion 9 and a truncated portion 10. The cylindrical portion 9 receives the lens 3. The truncated portion 10 receives the prism 5, the further prism 8 and the further lens 7.

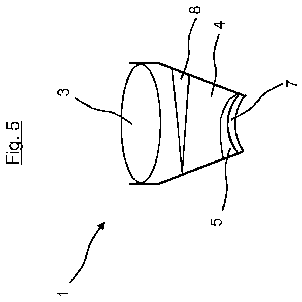

Figure 5 shows a device 1 for compensating physical and/or optical distortions of the human eye 6 according to a second embodiment. The device 1 differs from the device shown in figure 4 in that the prism 5 is directly attached to the further prism 8. That means, there is no distance between the prism 5 and the further prism 8 along a non-shown optical axis of the device 1.

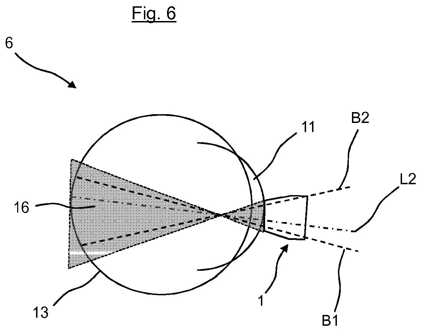

Figure 6 shows the inventive device 1 placed on a human eye 6. The device 1 can be formed as shown in figure 4 or figure 5. The device 1 is placed on the cornea 11 collinear to the optical axis L2 of the human eye 6. Further, several regions of the retina 13 are exposed with the first laser beam B1 and the second laser beam B2.

As is evident from figure 6 due to the provision of the optical components described above in the device 1 the optical zone 16 of the device 1 is greater than the field of view of the lens device 15 shown in figure 2. Further, the optical zone 16 is shifted in comparison to the optical zone 16 of the lens device 15 shown in figure 1.

Due to increasing of the optical zone 16 and shifting of the optical zone 16 to inferior and/or nasal and/or temporal parts of the human eye 6, it is not necessary to tilt the device 1 in order to treat interesting regions of the retina with at least one of the laser beams B1, B2. This is not necessary because all interesting regions of the retina 13 are arranged within the optical zone 16 of the device 1.

The provision of the optical components described additionally enable that laser spot images of uniform diameter and/or radiance are created as is shown in figure 7 as the distortions of the human eye 6 are compensated by the device 1.

Figure 7 shows the laser spots 19 visible with the device shown in figure 6. As can be seen from figure 7 both the laser spots 19 arranged in the upper arcade 17 as well as in the lower arcade 18 have a uniform diameter and/or radiance.

### Reference signs:

- 1: device
- 2: housing
- 3: lens
- 4: inner space
- 5: prism
- 6: human eye
- 7: further lens
- 8: further prism
- 9: cylindrical portion
- 10: truncated portion
- 11: cornea
- 12: eyeball
- 13: retina
- 14: fovea
- 15: lens device
- 16: optical zone
- 17: upper arcade
- 18: lower arcade
- 19: laser spot

- B1: first laser beam
- B2: second laser beam

- L1: visual axis
- L2: optical axis

- M1: centre of curvature of cornea
- M2: centre of eyeball
- M3: centre of pupil

## Claims

1. Device (1) for compensating physical and/or optical distortions of a human eye (6) comprising a housing (2) and a lens (3) arranged in an inner space (4) of the housing (2), the device (1) comprises a prism (5) arranged in the inner space (4) and in that the lens (3) is arranged at an end of the housing (2) such that laser light enters the device (1) via the lens (3), wherein the prism (5) is arranged between the lens (3) and a further lens (7), wherein the further lens (7) is arranged at a further end of the housing (2) such that it can be brought in direct contact with a cornea (11) of the human eye (6) and the laser light entering the device (1) leaves the device (1) via the further lens (7), wherein the lens (3) and the further lens (7) are aspheric lenses.

2. Device (1) according to claim 1 , **characterized in that**
a. the prism (5) and the further lens (7) and/or the lens (3) are arranged at distance from each other or
b. the prism (5) is attached to the further lens (7).

3. Device (1) according to one of the claims 1 to 2, **characterized in that** a further prism (8) is arranged in the inner space (4).

4. Device (1) according to claim 3, **characterized in that**
a. the prism (5) and the further prism (8) can be moved with respect to each other and/or **in that**
b. the prism (5) and the further prism (8) can be rotated with respect to each other.

5. Device (1) according to one of the claims 1 to 4, **characterized in that** the lens (3) and/or the prism (5) is releasably connected with the housing (2).

6. Device (1) according to one of the claims 1 to 5, **characterized in that** the further lens (7) and/or the further prism (8) and/or the lens (3) and/or the further lens (7) is releasably connected with the housing (2).

7. Device (1) according to one of claims 2 to 6, **characterized in that**
a. the prism (5) and the further prism (8) are connected together to be modular and/or **in that**
b. the prism (5) and the further lens (7) are connected together to be modular.

8. Device (1) according to one of the claims 1 to 7, **characterized in that**
a. the lens (3) can be exchanged by another lens from a plurality of another lenses, wherein the another lenses differ from each other in at least one optical property and/or **in that**
b. the prism (5) can be exchanged by another prism from a plurality of another prisms, wherein the another prisms differ from each other in at least one optical property.

9. Device (1) according to one of the claims 2 to 8, **characterized in that**
a. the further lens (7) can be exchanged by another further lens from a plurality of another further lenses, wherein the another further lenses differ from each other in at least one optical property and/or in that
b. the further prism (8) can be exchanged by another further prism (8) from a plurality of another further prisms, wherein the another further prisms differ from each other in at least one optical property.

10. Device (1) according to one of the claims 1 to 9, **characterized in that**
a. the housing (2) has a cylindrical portion (9) receiving the lens (3) and a truncated portion (10) receiving the prism (5) and/or **in that**
b. the device comprises an optical filter for blocking light of a specific wavelength and/or **in that**
c. the device comprises a filter coating for blocking light of a specific wavelength.

## Patentansprüche

1. Vorrichtung (1) zur Kompensation von physikalischen und/oder optischen Verzerrungen eines menschlichen Auges (6), umfassend ein Gehäuse (2) und eine in einem Innenraum (4) des Gehäuses (2) angeordnete Linse (3), wobei die Vorrichtung (1) ein in dem Innenraum (4) angeordnetes Prisma (5) umfasst und wobei die Linse (3) an einem Ende des Gehäuses (2) so angeordnet ist, dass Laserlicht über die Linse (3) in die Vorrichtung (1) eintritt, wobei das Prisma (5) zwischen der Linse (3) und einer weiteren Linse (7) angeordnet ist, wobei die weitere Linse (7) an einem weiteren Ende des Gehäuses (2) so angeordnet ist, dass sie in direkten Kontakt mit einer Hornhaut (11) des menschlichen Auges (6) gebracht werden kann und das in die Vorrichtung (1) eintretende Laserlicht die Vorrichtung (1) über die weitere Linse (7) verlässt, wobei die Linse (3) und die weitere Linse (7) asphärische Linsen sind.

2. Vorrichtung (1) nach Anspruch 1, **dadurch gekennzeichnet, dass**
a. das Prisma (5) und die weitere Linse (7) und/oder die Linse (3) in Abstand voneinander angeordnet sind oder
b. das Prisma (5) an der weiteren Linse (7) angebracht ist.

3. Vorrichtung (1) nach einem der Ansprüche 1 bis 2, **dadurch gekennzeichnet, dass** ein weiteres Prisma (8) in dem Innenraum (4) angeordnet ist.

4. Vorrichtung (1) nach Anspruch 3, **dadurch gekennzeichnet, dass**
a. das Prisma (5) und das weitere Prisma (8) in Bezug zueinander bewegt werden können, und/oder dadurch, dass
b. das Prisma (5) und das weitere Prisma (8) in Bezug zueinander gedreht werden können.

5. Vorrichtung (1) nach einem der Ansprüche 1 bis 4, **dadurch gekennzeichnet, dass** die Linse (3) und/oder das Prisma (5) lösbar mit dem Gehäuse (2) verbunden ist.

6. Vorrichtung (1) nach einem der Ansprüche 1 bis 5, **dadurch gekennzeichnet, dass** die weitere Linse (7) und/oder das weitere Prisma (8) und/oder die Linse (3) und/oder die weitere Linse (7) lösbar mit dem Gehäuse (2) verbunden ist.

7. Vorrichtung (1) nach einem der Ansprüche 2 bis 6, **dadurch gekennzeichnet, dass**
a. das Prisma (5) und das weitere Prisma (8) miteinander verbunden sind, um modular zu sein, und/oder dadurch, dass
b. das Prisma (5) und die weitere Linse (7) miteinander verbunden sind, um modular zu sein.

8. Vorrichtung (1) nach einem der Ansprüche 1 bis 7, **dadurch gekennzeichnet, dass**
a. die Linse (3) durch eine andere Linse aus einer Vielzahl von anderen Linsen ersetzt werden kann, wobei sich die anderen Linsen in mindestens einer optischen Eigenschaft voneinander unterscheiden, und/oder dadurch, dass
b. das Prisma (5) durch ein anderes Prisma aus einer Vielzahl von anderen Prismen ersetzt werden kann, wobei sich die anderen Prismen in mindestens einer optischen Eigenschaft voneinander unterscheiden.

9. Vorrichtung (1) nach einem der Ansprüche 2 bis 8, **dadurch gekennzeichnet, dass**
a. die weitere Linse (7) durch eine andere weitere Linse aus einer Vielzahl von anderen weiteren Linsen ersetzt werden kann, wobei sich die anderen weiteren Linsen in mindestens einer optischen Eigenschaft voneinander unterscheiden, und/oder dadurch, dass
b. das weitere Prisma (8) durch ein anderes weiteres Prisma (8) aus einer Vielzahl von anderen weiteren Prismen ersetzt werden kann, wobei sich die anderen weiteren Prismen in mindestens einer optischen Eigenschaft voneinander unterscheiden.

10. Vorrichtung (1) nach einem der Ansprüche 1 bis 9, **dadurch gekennzeichnet, dass**
a. das Gehäuse (2) einen die Linse (3) aufnehmenden zylindrischen Abschnitt (9) und einen das Prisma (5) aufnehmenden abgestumpften Abschnitt (10) aufweist, und/oder dadurch, dass
b. die Vorrichtung einen optischen Filter zum Blockieren von Licht einer bestimmten Wellenlänge umfasst und/oder dadurch, dass
c. die Vorrichtung eine Filterbeschichtung zum Blockieren von Licht einer spezifischen Wellenlänge umfasst.

## Revendications

1. Dispositif (1) de compensation de distorsions physiques et/ou optiques d'un œil humain (6) comprenant un boîtier (2) et une lentille (3) agencée dans un espace intérieur (4) du boîtier (2), le dispositif (1) comprend un prisme (5) agencé dans l'espace intérieur (4) et en ce que la lentille (3) est agencée à une extrémité du boîtier (2) de sorte qu'une lumière laser entre dans le dispositif (1) par l'intermédiaire de la lentille (3), dans lequel le prisme (5) est agencé entre la lentille (3) et une lentille supplémentaire (7), dans lequel la lentille supplémentaire (7) est agencée à une extrémité supplémentaire du boîtier (2) de sorte qu'elle puisse être mise en contact direct avec une cornée (11) de l'œil humain (6) et la lumière laser entrant dans le dispositif (1) quitte le dispositif (1) par l'intermédiaire de la lentille supplémentaire (7), dans lequel la lentille (3) et la lentille supplémentaire (7) sont des lentilles asphériques.

2. Dispositif (1) selon la revendication 1, **caractérisé en ce que**
a. le prisme (5) et la lentille supplémentaire (7) et/ou la lentille (3) sont agencés à distance l'un de l'autre ou
b. le prisme (5) est fixé à la lentille supplémentaire (7).

3. Dispositif (1) selon l'une des revendications 1 à 2, **caractérisé en ce qu'**un prisme supplémentaire (8) est agencé dans l'espace intérieur (4).

4. Dispositif (1) selon la revendication 3, **caractérisé en ce que**
a. le prisme (5) et le prisme supplémentaire (8) peuvent être déplacés l'un par rapport à l'autre et/ou **en ce que**
b. le prisme (5) et le prisme supplémentaire (8) peuvent être tournés l'un par rapport à l'autre.

5. Dispositif (1) selon l'une des revendications 1 à 4, **caractérisé en ce que** la lentille (3) et/ou le prisme (5) est relié(s) de manière amovible au boîtier (2).

6. Dispositif (1) selon l'une des revendications 1 à 5, **caractérisé en ce que** la lentille supplémentaire (7) et/ou le prisme supplémentaire (8) et/ou la lentille (3) et/ou la lentille supplémentaire (7) est reliés de manière amovible au boîtier (2).

7. Dispositif (1) selon l'une des revendications 2 à 6, **caractérisé en ce que**
a. le prisme (5) et le prisme supplémentaire (8) sont reliés ensemble pour être modulaires et/ou **en ce que**
b. le prisme (5) et la lentille supplémentaire (7) sont reliés ensemble pour être modulaires.

8. Dispositif (1) selon l'une des revendications 1 à 7, **caractérisé en ce que**
a. la lentille (3) peut être échangée par une autre lentille d'une pluralité d'autres lentilles, lesdites autres lentilles différant les unes des autres par au moins une propriété optique et/ou **en ce que**
b. le prisme (5) peut être échangé par un autre prisme d'une pluralité d'autres prismes, lesdits autres prismes différant les uns des autres par au moins une propriété optique.

9. Dispositif (1) selon l'une des revendications 2 à 8, **caractérisé en ce que**
a. la lentille supplémentaire (7) peut être échangée par une autre lentille supplémentaire d'une pluralité d'autres lentilles supplémentaires, lesdites autres lentilles supplémentaires différant les unes des autres par au moins une propriété optique et/ou **en ce que**
b. le prisme supplémentaire (8) peut être échangé par un autre prisme supplémentaire (8) d'une pluralité d'autres prismes supplémentaires, lesdits autres prismes supplémentaires différant les uns des autres par au moins une propriété optique.

10. Dispositif (1) selon l'une quelconque des revendications 1 à 9, **caractérisé en ce que**
a. le boîtier (2) comporte une partie cylindrique (9) recevant la lentille (3) et une partie tronconique (10) recevant le prisme (5) et/ou **en ce que**
b. le dispositif comprend un filtre optique pour bloquer une lumière d'une longueur d'onde spécifique et/ou **en ce que**
c. le dispositif comprend un revêtement filtrant pour bloquer une lumière d'une longueur d'onde spécifique.
